(19) Europäisches Patentamt
European Patent Office
Office européen des brevets

(11) Veröffentlichungsnummer: **0 079 515**
**A2**

(12) **EUROPÄISCHE PATENTANMELDUNG**

(21) Anmeldenummer: **82110075.7**

(22) Anmeldetag: **02.11.82**

(51) Int. Cl.[3]: **C 07 C 127/22**

(30) Priorität: **14.11.81 DE 3145327**

(43) Veröffentlichungstag der Anmeldung: **25.05.83 Patentblatt 83/21**

(84) Benannte Vertragsstaaten: **BE CH DE FR GB LI**

(71) Anmelder: **BASF Aktiengesellschaft**
**Carl-Bosch-Strasse 38**
**D-6700 Ludwigshafen(DE)**

(72) Erfinder: **Schwendemann, Volker, Dr.**
**Hauptstrasse 64a**
**D-6901 Wiesenbach(DE)**

(54) **Verfahren zur Herstellung von N,N'-disubstituierten Allophansäureestern.**

(57) Verfahren zur Herstellung von N,N'-disubstituierten Allophansäureestern durch Umsetzung von aromatischen Chlorameisensäureestern mit N,N'-disubstituierten Harnstoffen bei 20 bis 160°C ohne Zusatz säurebindender Mittel.

Die nach dem Verfahren der Erfindung herstellbaren N,N'-disubstituierten Allophansäureester sind wertvolle Ausgangsstoffe für die Herstellung von Pflanzenschutzmitteln, Schädlingsbekämpfungsmitteln, Farbstoffen, Kunstharzen und Kunststoffen, Textilhydrophobierungsmitteln, Waschmitteln, Bleichmitteln und Klebstoffen.

EP 0 079 515 A2

Croydon Printing Company Ltd

Verfahren zur Herstellung von N,N'-disubstituierten Allophansäureestern

Die Erfindung betrifft ein Verfahren zur Herstellung von N,N'-disubstituierten Allophansäureestern durch Umsetzung von aromatischen Chlorameisensäureestern mit N,N'-disubstituierten Harnstoffen in bestimmten Molverhältnissen bei 80 bis 200°C ohne Zusatz säurebindender Mittel.

Es ist bekannt, Allophansäureester durch Addition von Cyansäure an Alkoholen darzustellen (Houben-Weyl, Methoden der Organischen Chemie, Band 8, Seite 204 (1952)).

Ebenso ist es bekannt, Allophansäureester durch Addition von Isocyanaten bzw. Cyansäure an Carbamate herzustellen. Die dabei in der Technik auftretenden Probleme ergeben sich vor allem bei der Verwendung bzw. bei Manipulationen mit den äußerst toxischen Isocyanaten und Cyansäure. Die Reaktion von Isocyanaten mit Carbamaten wird zur Erreichung einer guten Ausbeute unter der katalytischen Wirkung von Basen oder Schwermetallsalzen durchgeführt (J. Org. Chem., Band 26, Seite 3 004 (1961)). Diese Bedingungen führen jedoch häufig zu einer Trimerisierung des Isocyanats bzw. der Isocyansäure (US-PS 3 372 180, Beispiel 1).

Es ist weiterhin bekannt (Houben-Weyl, Methoden der Organischen Chemie, Band 8, Seite 206 (1952)), Allophansäureester durch Umsetzung von Allophansäurechloriden mit Hydroxyverbindungen darzustellen. Die hierfür notwendigen Allophansäurechloride sind jedoch nur schwer zugänglich und in mäßigen Ausbeuten aus Harnstoffen und Phosgen herzustellen.

Die Umsetzung von Chlorameisensäureester mit Dialkylharnstoffen zu N,N'-Dialkylallophanaten ist aus z.B. Houben-Weyl, Methoden der Organischen Chemie, Band 8,

Mu/P

Seite 205 (1952) bekannt. Bei Durchführung der Reaktion wird jedoch der Dialkylharnstoff mit mindestens 1 Mol Überschuß über die stöchiometrische Menge eingesetzt, um den bei der Reaktion entstehenden Chlorwasserstoff zu binden. Selbst dann liegen die Ausbeuten nur unter 65 %.

Es wurde nun gefunden, daß man N,N'-disubstituierte Allophansäureester der Formel

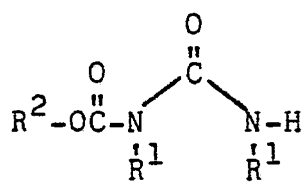 I,

worin die einzelnen Reste $R^1$ gleich oder verschieden sein können und einen aliphatischen Rest bedeuten, und $R^2$ einen aromatischen Rest bezeichnet, durch Umsetzung von Chlorameisensäureestern mit Harnstoff vorteilhaft erhält, wenn man einen aromatischen Chlorameisensäureester der Formel

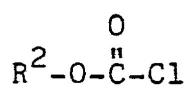 II,

worin $R^2$ die vorgenannte Bedeutung besitzt, mit N,N'-disubstituierten Harnstoffen der Formel

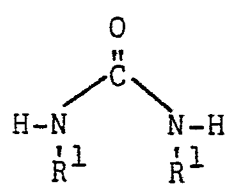 III,

worin $R^1$ die vorgenannte Bedeutung besitzt, in einem Verhältnis von weniger als 2 Mol Ausgangsstoff III je Mol Ausgangsstoff II bei einer Temperatur von 80 bis 200 °C ohne Zusatz säurebindender Mittel umsetzt.

Die Reaktion kann beispielsweise im Falle der Verwendung von N,N'-Dimethylharnstoff und Chlorameisensäurephenylester durch die folgenden Formeln wiedergegeben werden:

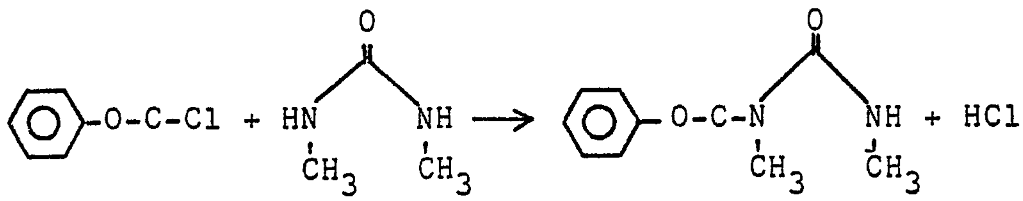

Im Vergleich zu den bekannten Verfahren liefert das erfindungsgemäße Verfahren auf einfacherem und wirtschaftlicherem Wege N,N'-disubstituierte Allophansäureester in besserer Ausbeute und Reinheit. Die anfallenden Endstoffe I weisen in der Regel eine Reinheit von über 95 % auf und können durch bekannte Methoden weitergereinigt oder ohne weitere Reinigung verwendet werden. Das Arbeiten mit den toxisch äußerst bedenklichen Isocyanaten bzw. Cyansäure wird vermieden. Es werden keine Katalysatorzusätze benötigt. Ein umweltbelastender Salzanfall wird vermieden. Alle diese vorteilhaften Ergebnisse sind im Hinblick auf den Stand der Technik überraschend. Man hätte angesichts der geringen Basenmenge im Reaktionsgemisch eine schlechtere Ausbeute bzw. ein heterogenes Gemisch verschiedener Nebenstoffe und Zersetzungsprodukte erwartet.

Bevorzugte Ausgangsstoffe II, III und dementsprechend bevorzugte Endstoffe I sind solche, in deren Formeln die einzelnen Reste $R^1$ gleich oder verschieden sein können und einen Alkylrest mit 1 bis 7, insbesondere 1 bis 4 Kohlenstoffatomen oder einen Alkenylrest mit 2 bis 7, insbesondere 2 bis 4 Kohlenstoffatomen bedeuten, und $R^2$ einen Phenylrest, der noch durch aliphatische Reste, insbesondere Alkylreste mit 1 bis 10 Kohlenstoffatomen, Alkoxygruppen, insbesondere mit 1 bis 4 Kohlenstoffatomen, Fluoratome, Chloratome, Nitrogruppen substituiert sein kann. Die vorgenannten Reste können noch durch unter den Reaktionsbedingungen inerte Gruppen und/oder Atome, z.B. Nitrogruppen, Alkylgruppen, Alkoxygruppen mit jeweils 1 bis 4 Kohlenstoffatomen, substituiert sein.

Die Ausgangsstoffe werden in stöchiometrischer Menge oder im Überschuß, in einem Verhältnis von weniger als 2 Mol Ausgangsstoff III je Mol Ausgangsstoff II, vorteilhaft 0,8 bis 1,8, insbesondere 0,9 bis 1,2 Mol Ausgangsstoff III je Mol Ausgangsstoff II umgesetzt.

So sind beispielsweise folgende Ausgangsstoffe II geeignet: unsubstituierter Chlorameisensäurephenylester; durch 1 oder 2 Methyl-, Ethyl-, Propyl-, Isopropyl-, Butyl-, Isobutyl-, tert.-Butyl-, sek.-Butyl-, Pentyl-, Hexyl-, Heptyl-, Octyl-, Nonyl-, Decyl-, Allyl-, Nitro-, Methoxy-, Ethoxy-, Propoxygruppen, Fluoratome, Chloratome am Phenylkern des Phenols in 2'-, 3'-, 4'-Stellung einfach oder in 2',6'-, 2',3'-, 2',4'-, 2',5'-, 3',5'-, 4',5'-Stellung gleich oder unterschiedlich zweifach substituierte Phenylester der Chlorameisensäure.

So kommen folgende Ausgangsstoffe III in Betracht: N,N'-Dimethylharnstoff; die homologen N,N'-Diethyl-, N,N'-Dipropyl-, N,N'-Diisopropyl-, N,N'-Dibutyl-, N,N'-Diallyl-, N,N'-Diisobutyl-, N,N'-Di-tert.-butyl-, N,N'-Di-sek.-butyl-, N-Methyl-N'-Ethylverbindungen des Harnstoffs.

Die Umsetzung wird bei einer Temperatur von 20 bis 200°C, vorzugsweise von 100 bis 150°C, mit Unterdruck, Überdruck oder drucklos, diskontinuierlich oder kontinuierlich, durchgeführt. Zweckmäßig verwendet man unter den Reaktionsbedingungen inerte, organische Lösungsmittel. Als Lösungsmittel kommen z.B. in Frage: aromatische Kohlenwasserstoffe, z.B. Toluol, Ethylbenzol, o-, m-, p-Xylol, Isopropylbenzol, Methylnaphthalin; Halogenkohlenwasserstoffe, insbesondere Brom- und Chlorkohlenwasserstoffe, z.B. Tetrachlorethylen, 1,1,2,2- oder 1,1,1,2-Tetrachlorethan, Amylchlorid, Cyclohexylchlorid, 1,2-Dichlorpropan, Methylenchlorid, Dichlorbutan, Isopropylbromid, n-Propylbromid, Butylbromid, Chloroform, Bromoform, Ethyljodid, Propyljodid, Chlornaphthalin, Dichlornaphthalin, Tetrachlorkohlenstoff, 1,1,1- oder 1,1,2-Trichlorethan, Trichlorethylen, Pentachlorethan, 1,2-Dichlorethan, 1,1-Dichlorethan, n-Propylchlorid, 1,2-cis-Dichlorethylen, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; Ketone wie Methylethylketon, Aceton, Diisopropylketon, Diethylketon, Methylisobutylketon, Mesityloxid, Acetophenon, Cyclohexanon, Ethylisoamylketon, Diisobutylketon, Methylcyclohexanon, Dimethylcyclohexanon; Ester wie Methylacetat, n-Propylacetat, Methylpropionat, Butylacetat, Ethylformiat, Phthalsäuremethylester, Benzoesäuremethylester, Essigester, Phenylacetat; Nitrokohlenwasserstoffe wie Nitromethan, Nitroethan, Nitrobenzol, o-, m-, p-Chlornitrobenzol, o-Nitrotoluol; Sulfoxide wie Dimethylsulfoxid, Diethylsulfoxid, Dimethylsulfon, Diethylsulfon, Methylethylsulfon, Tetramethylensulfon; aliphatische oder cycloaliphatische Kohlenwasserstoffe, z.B. Pentan, Heptan, α-Pinen, Pinan, Nonan, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; und entsprechende Gemische. Zweckmäßig verwendet man das Lösungsmittel in einer Menge von 200 bis 10 000 Gewichtsprozent, vorzugsweise von 200 bis 1 000 Gewichtsprozent, bezogen auf Ausgangsstoff II.

Die Reaktion kann wie folgt durchgeführt werden: Ein Gemisch von Ausgangsstoff II und Ausgangsstoff III und zweckmäßig Lösungsmittel wird während 5 bis 30 Stunden bei der Reaktionstemperatur gehalten. Dann wird aus dem Reaktionsgemisch der Endstoff in üblicher Weise, z.B. durch Destillation oder Kristallisation, abgetrennt. In einer bevorzugten Ausführungsform wird in einem inerten Lösungsmittel der Dialkylharnstoff vorgelegt und anschließend mit dem Chlorameisensäurearylester zur Reaktion gebracht. Nach beendeter Chlorwasserstoffabspaltung wird der entstandene Endstoff I durch Absaugen oder Einengen der Reaktionslösung erhalten.

Ebenso kann die Reaktion durch gleichzeitige Zugabe der Ausgangsstoffe II und III zu dem Verdünnungsmittel oder durch Zugabe des Ausgangsstoffs III zu der Lösung des Ausgangsstoffs II in dem Verdünnungsmittel erfolgen. Durch Einblasen eines Inertgasstroms in die Reaktionslösung kann der Chlorwasserstoff beschleunigt ausgetrieben werden.

Die nach dem Verfahren der Erfindung herstellbaren N,N'-disubstituierten Allophansäureester sind wertvolle Ausgangsstoffe für die Herstellung von Pflanzenschutzmitteln, Schädlingsbekämpfungsmitteln, Farbstoffen, Kunstharzen und Kunststoffen, Textilhydrophobierungsmitteln, Waschmitteln, Bleichmitteln und Klebstoffen. Ihre thermische Zersetzung zu aliphatischen Isocyanaten kann in der Regel ohne weitere Reinigung durchgeführt werden, z.B. in organischen Carbonaten bei 150 bis 400°C. Insbesondere sind die Umsetzungen der so erhaltenen Isocyanate zu Urethanen, z.B. für die Verwendung als Svhaumstoffe oder hochmolekulare Überzüge mit hoher Flexibilität, oder Harnstoffe von Bedeutung. Bezüglich der Verwendung wird auf vorgenannte Veröffentlichungen und Ullmanns Encyklopädie der technischen Chemie, Band 9, Seiten 11, 12, 404, und Band 17, Seite 204, verwiesen.

Die in den folgenden Beispielen aufgeführten Teile bedeuten Gewichtsteile.

Beispiel 1

184 Teile N,N'-Dimethylharnstoff werden in 800 Teilen Toluol vorgelegt und bei 23°C werden 340 Teile Chlorameisensäure-m-kresylester zugegeben. Dann wird das Gemisch 24 Stunden unter Durchfluß von Stickstoff durch die Lösung am Rückfluß (111°C) erhitzt und anschließend zur Trockne eingeengt. Man wäscht mit Wasser und erhält nach dem Trocknen 420 Teile (95 % der Theorie) N,N'-Dimethylallophansäure-m-kresylester vom Fp 91°C.

Beispiel 2

352 Teile N,N'-Dimethylharnstoff werden in 2 000 Teilen n-Octan vorgelegt und zum Rückfluß (126°C) erhitzt. Dann werden unter Rückfluß 684 Teile Chlorameisensäure-p-kresylester zugegeben und das Gemisch unter Durchleiten von Stickstoff noch 6 Stunden am Rückfluß gekocht. Nach dem Abkühlen saugt man den entstandenen Festkörper ab. Man erhält 824 Teile (93 % der Theorie) N,N'-Dimethylallophansäure-p-kresylester vom Fp 92°C.

Beispiel 3

352 Teile N,N'-Dimethylharnstoff werden in 800 Teilen Toluol vorgelegt und bei 23°C 625 Teile Chlorameisensäurephenylester zugegeben. Man erhitzt das Gemisch 24 Stunden am Rückfluß (111°C) und destilliert anschließend das Lösungsmittel ab. Die heiße Schmelze wird auf Wasser gegossen und der entstandene Festkörper abfiltriert. Man erhält 773 Teile (93 % der Theorie) N,N'-Dimethylallophansäurephenylester vom Fp 97 bis 98°C.

Beispiel 4

352 Teile N,N'-Dimethylharnstoff werden in 2 000 Teilen n-Octan gelöst und bei Rückflußtemperatur (126°C) werden 625 Teile Chlorameisensäurephenylester zugegeben. Man erhitzt das Gemisch noch 12 Stunden unter Rückfluß, läßt abkühlen und saugt den kristallinen Festkörper ab. Man erhält 770 Teile (93 % der Theorie) N,N'-Dimethylallophansäurephenylester vom Fp 96 bis 98°C.

Beispiel 5

352 Teile N,N'-Dimethylharnstoff werden in 1 500 Teilen Testbenzin (Kp 100 bis 140°C) gelöst und bei Rückflußtemperatur (110°C) 625 Teile Chlorameisensäurephenylester zugegeben. Man erhitzt das Gemisch noch 12 Stunden unter Rückfluß, läßt abkühlen und saugt den kristallinen Festkörper ab. Man erhält 760 Teile (90,5 % der Theorie) N,N'-Dimethylallophansäurephenylester vom Fp 97 bis 98°C.

Beispiel 6

44 Teile N,N'-Dimethylharnstoff in 300 Teilen n-Octan werden zum Rückfluß (126°C) erhitzt und 141 Teile Chlorameisensäure-p-nonylphenylester zugegeben. Man erhitzt das Gemisch 24 Stunden unter Einleiten von Stickstoff in die Lösung am Rückfluß und erhält nach Einengen der Lösung 160 Teile (96 % der Theorie) N,N'-Dimethylallophansäure-p--nonylphenylester.

NMR ($CDCl_3$) $\delta$ = 8,4 [1H] t
7,0-7,5 [4H] m
3,4 [3H] s
2,8 [3H] d
0,5-1,8 [19H] m

Beispiel 7

39 Teile N-Methyl-N'-isobutylharnstoff werden in 200 Teilen n-Octan vorgelegt und unter Rückfluß 85 Teile Chlorameisensäure-p-nonylphenylester zugegeben. Man erhitzt das Gemisch 30 Stunden am Rückfluß (126°C) und engt anschließend die Lösung ein. Man erhält 109 Teile (96 % der Theorie) N-Methyl-N'-isobutylallophansäure-p-nonylphenylester.

NMR ($CDCl_3$) $\delta$ = 8,6 [1H] t
= 7,0-7,5 [4H] m
= 3,5 [3H] s
= 3,2 [2H] dd
= 1,8 [1H] m
= 1,0 [6H] d
= 0,5-2,0 [19H] m

## Patentanspruch

Verfahren zur Herstellung von N,N'-disubstituierten Allophansäureestern der Formel

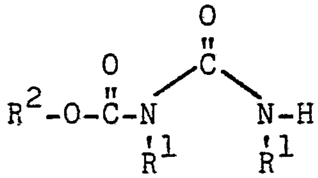

I,

worin die einzelnen Reste $R^1$ gleich oder verschieden sein können und einen aliphatischen Rest bedeuten, und $R^2$ einen aromatischen Rest bezeichnet, durch Umsetzung von Chlorameisensäureestern mit Harnstoff, dadurch gekennzeichnet, daß man einen aromatischen Chlorameisensäureester der Formel

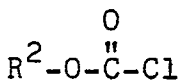

II,

worin $R^2$ die vorgenannte Bedeutung besitzt, mit N,N'-disubstituierten Harnstoffen der Formel

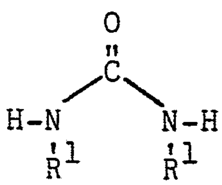

III,

worin $R^1$ die vorgenannte Bedeutung besitzt, in einem Verhältnis von weniger als 2 Mol Ausgangsstoff III je Mol Ausgangsstoff II bei einer Temperatur von 80 bis 200°C ohne Zusatz säurebindender Mittel umsetzt.